# EUROPEAN PATENT APPLICATION

(11) **EP 3 974 013 A1**
(43) Date of publication of application: **30.03.2022**
(21) Application number: 21000265.5
(22) Date of filing: 22.09.2021
(51) Int. Cl.: A61M 15/00

(54) **INHALER FOR DELIVERING MEDICINES**

(30) Priority: 24.09.2020 PL 43543420
(71) Applicant: Polfarmex S.A., 99-300 Kutno (PL)
(72) Inventor: Prus, Artur, Warsaw (PL)

(57) **Abstract**

An inhaler for administering a medicament in the form of dry powder from blister packs seated on a conveyor, wherein the administration of the medicament involves simultaneous movement of two geometrically identical blister packs (01) by a specified step determined by the distance between the pockets (01.1) with the medicament in each blister pack strip, and positioning the pockets centrally to the axes of air channels (05.1) in the body (05), each of the two blister packs (01) being mounted in their corresponding conveyors (06) and (07), coupled with each other by means of gear wheels (06.2), (09), (11), (13), latch elements (09.1), (05.2) connected to each other so as to prevent the movement of only one blister pack, and so that both blister packs would move by a constant identical value.

## Description

The object of the invention is an inhaler allowing for the dispensing of a medicament from a blister pack by inhalation. In known solutions, blister packs are wound into a loose roll and placed in a chamber. Their movement is caused by the rotations of a rotor and a conveyor. The conveyor is constructed in such a manner that it has only 4 to 8 slots for blister pack pockets, and it only serves the function of an element conveying the layer of blister pack pockets to a pulling roller. The layer of blister pack pockets is further wound onto the roller and stored in a separate chamber.

The European patent with the publication number EP 2432530 describes an inhaler for delivering a dry-powder medicament to a patient from an open blister pocket of a blister pack, wherein the blister pack comprising a plurality of blister pockets spaced apart in the length direction of the pack, each of them containing a measured dose of the medicament, the inhaler comprising: a housing enclosing used and unused portions of the blister pack, and a medicament dispensing mechanism for opening the blister pockets of the blister pack; and a manifold through which air can be drawn in during use, the manifold comprising an air inlet for receiving external air, at least one medicament aperture for communicating with the open pocket of the blister pack to enable entrainment of the medicament by the air drawn through the manifold, and an air outlet for the delivery of the entrained medicament to the patient, the inhaler being characterised in that the manifold and a part of the housing are provided as a unitary moulded plastics component, and wherein the unitary component defines, in use, at least a portion of the outer surface of the inhaler, in which outer surface the air inlet is arranged.

The inhaler disclosed in US patent no. 5,590,645 comprises a medicament-dispensing mechanism, which provides peeling parts of a lid sheet off a base sheet of a blister pack, due to which a blister pocket is opened during every activation of the inhaler. This mechanism comprises means for sliding the blister pack in order to move an open pocket to a position aligned with the medicament openings made in the manifold of the inhaler, the manifold being connected in a manner enabling the flow of fluid to a mouthpiece, by means of which the patient can perform the inhalation. The unused portion of the blister pack and the used portions of the base sheet and the lid sheet are stored inside the body of the inhaler. In use of the inhaler described above, the patient exposes the mouthpiece by rotating the cover relative to the body of the inhaler, upon which they use an activating element to activate the medicament-dispensing mechanism. Subsequently, the patient performs inhalation by means of the mouthpiece, drawing air from inside the body of the inhaler through the manifold. The stream of air flowing through the mouthpiece and the manifold entrains the medicament in the form of dry powder placed in the open blister pocket, this medicament being delivered to the patient in the form of an aerosol. Subsequently, the activating element and the cover of the inhaler are placed in their initial positions, which is intended to prepare the medicament-dispensing mechanism for another use.

Although inhalers for dry powder known from prior art provide improved invariability of the size of doses administered to a patient, there is still a need for inhalers providing improved invariability of the dose size and safety of administering the medicament. At the same time, there is a need for inhalers allowing for minimising the risk of accidental exposure of the mouthpiece. In addition, a common problem in dry powder inhalers with tearable blister packs is the smoothness of operation and the rather considerable force needed to activate the mechanism.

The essence of the invention is an inhaler for administering a medicament in the form of dry powder from blister pockets, with simultaneous movement of two geometrically identical blister packs by a specified step determined by the distance between the pockets with the medicament in each blister pack strip, and the positioning of the pockets centrally to the axes of air channels in the body. Each of the two blister packs is mounted in their corresponding conveyors, coupled with each other by means of gear wheels, latch elements connected to each other so as to prevent the movement of only one blister pack, and so that both blister packs would move by a constant identical value.

The inhaler for administering a medicament in the form of dry powder from blister packs seated on a conveyor, wherein the lidding layer of the blister pack is torn before an air chamber and the medicament in the form of powder is transported by breathing in through the mouth contacting a mouthpiece, causing suction of the air and flow through an open blister pack pocket, is characterised in that the operation of the inhaler involves simultaneous movement of two geometrically identical blister packs by a specified step determined by the distance between the pockets with the medicament in each blister pack strip, and positioning the pockets centrally to the axes of air channels in the body, each of the two blister packs being mounted in their corresponding conveyors, coupled with each other by means of gear wheels, latch elements connected to each other so as to prevent the movement of only one blister pack, and so that both blister packs would move by a constant identical value. Preferably, the individual parts of the mechanism except the upper conveyor of the second blister pack are seated in a lower housing and covered by a body, the conveyor of the second blister pack being placed above the body and the whole being covered by an upper housing, the blister packs being mounted on two separate conveyors: lower and upper, having the shape of rings placed one above the other and connected to each other by means of a system of slots and wedges. Preferably, each of the blister pack pockets has its corresponding, fixed pocket in their corresponding conveyors, the lidding layer of the blister pack being torn right before the air chamber of the body, each time exposing one pocket from each blister pack, the lidding layer travelling in a fixed position on the conveyor, attached to a mounting slit in the lower conveyor and correspondingly in the upper conveyor, rotating with each subsequent dosing by a constant angle of rotation, whereas the pocket foil is mounted by its end on the pin of a rotor hook, the pocket foil becoming wound on the rotor by a constant value adjusted by flexible wings and the selected gear ratio of the gear wheels, whereas the rotor is seated on the rotor hook, which in turn is seated in a rotor wheel, the rotor gear wheel and the conveyor being coupled with each other via an intermediate wheel (04), a latch wheel (16), a latch, a latch drum, and they are driven by a driving roller, the driving roller, via the lower and upper catcher, transmitting the rotation from a slide (04) connected to the driving roller by recesses, the air channels of the body being connected to the air channels in the upper and lower housing, and, via channels formed by connecting the conveyors to each other, they are connected to the channel of the mouthpiece. Preferably, each of the blister packs 01 has 30 pockets with the medicament.

Preferably, a locking lever has been introduced in the upper housing as an elastic element for activating the dosing mechanism.

Preferably, a dose counter is placed on the conveyor, each of the slots on the conveyor being described by a printed number, the number being visible through a window in the upper housing.

Preferably, a high gear ratio is used on the gear wheels, causing the force necessary to activate the mechanism to be minimised, the positioning of the blister packs one above the other allowing for minimising the number of parts of the mechanism, mirror image parts being absent.

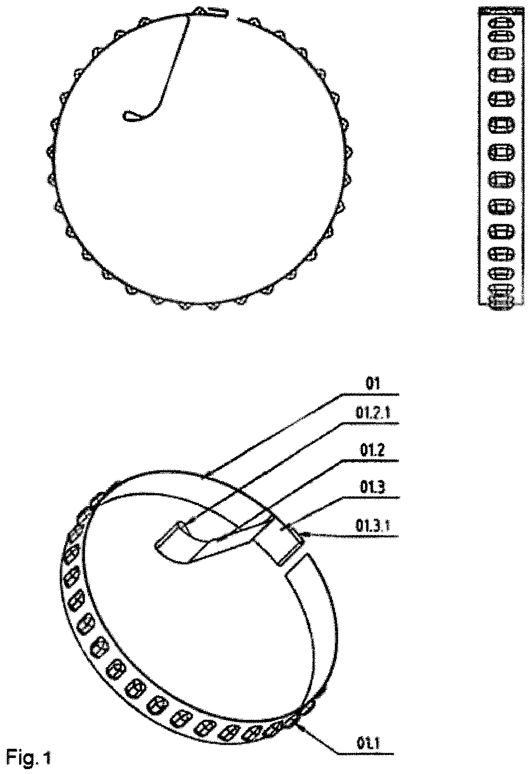

Fig. 1 is a general view of the blister pack showing the key elements.

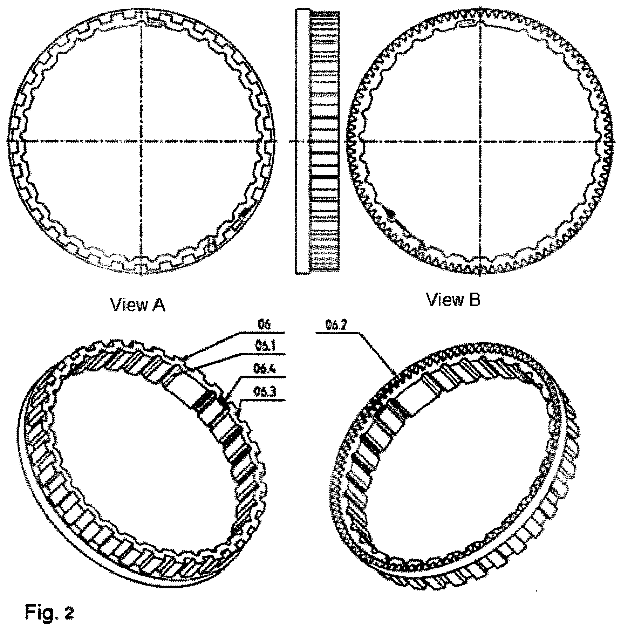

Fig. 2 is a general view of the conveyor showing the key elements.

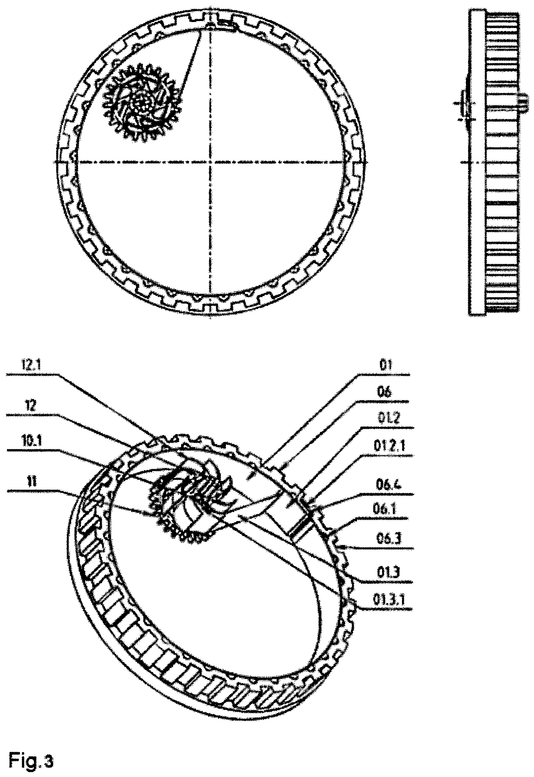

Fig. 3 is a general view of the manner of mounting the blister pack on the transport elements, also showing the key elements.

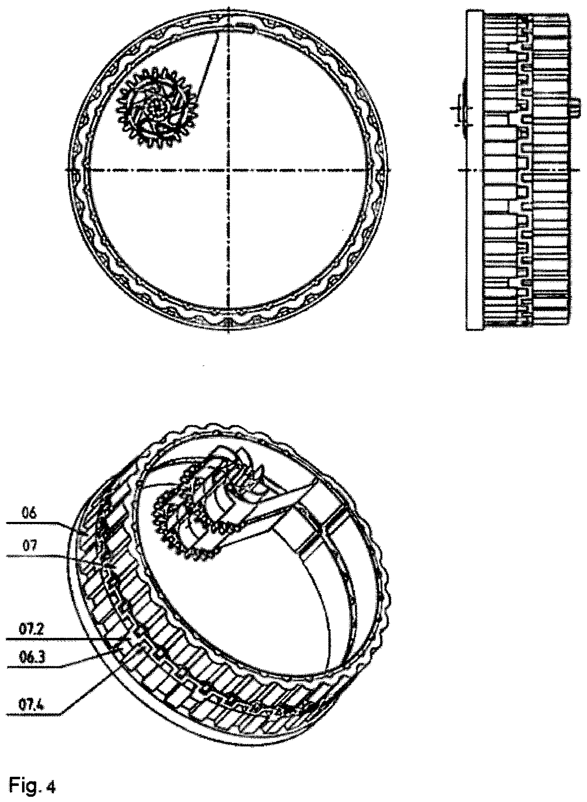

Fig. 4 is a general view of the manner of mounting two blister packs on the transport elements, also showing the key elements.

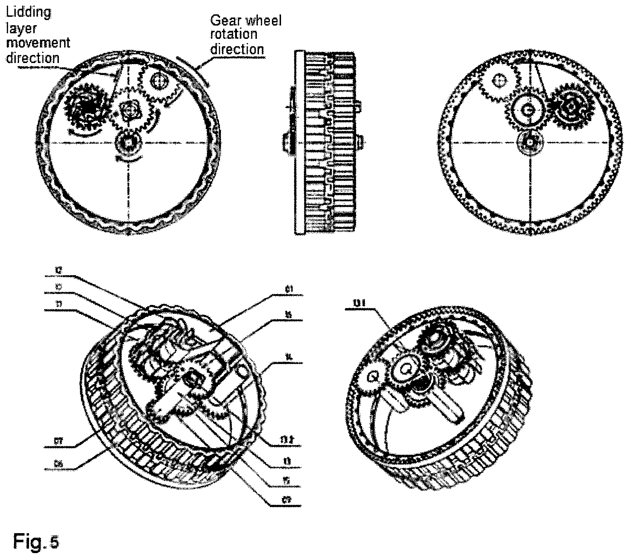

Fig. 5 presents the mechanism, the direction of rotations for the gear wheels of the mechanism and of movement for the layer of blister packs.

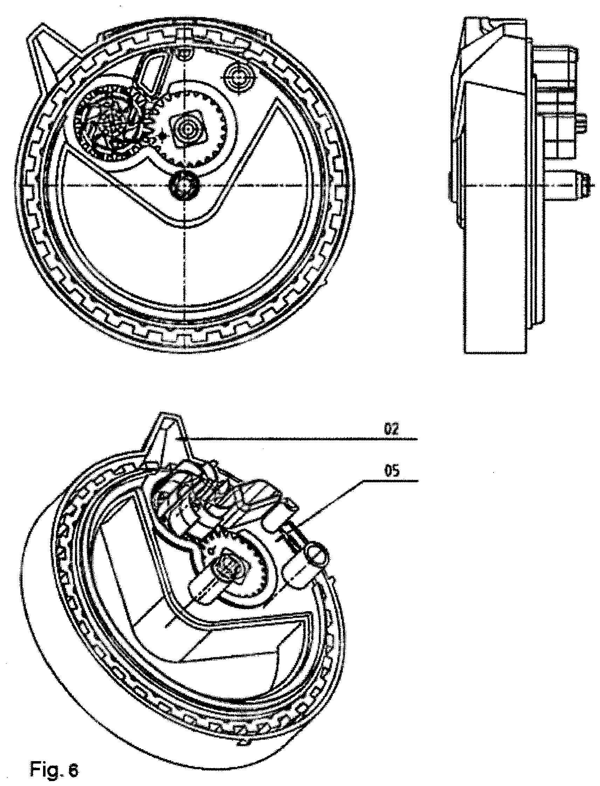

Fig. 6 is a general view of the elements of the mechanism enclosed in the housing and covered by the body, also showing the key elements. Not presenting the second conveyor-blister pack set.

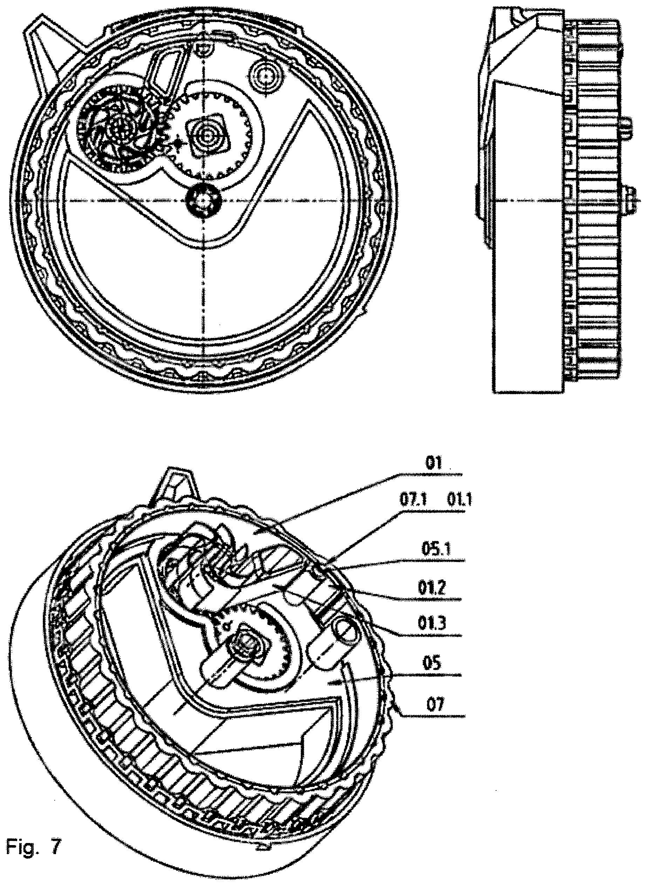

Fig. 7 is a general view of the elements of the mechanism enclosed in the housing and covered by the body, also showing the key elements. Presenting the second conveyor-blister pack set.

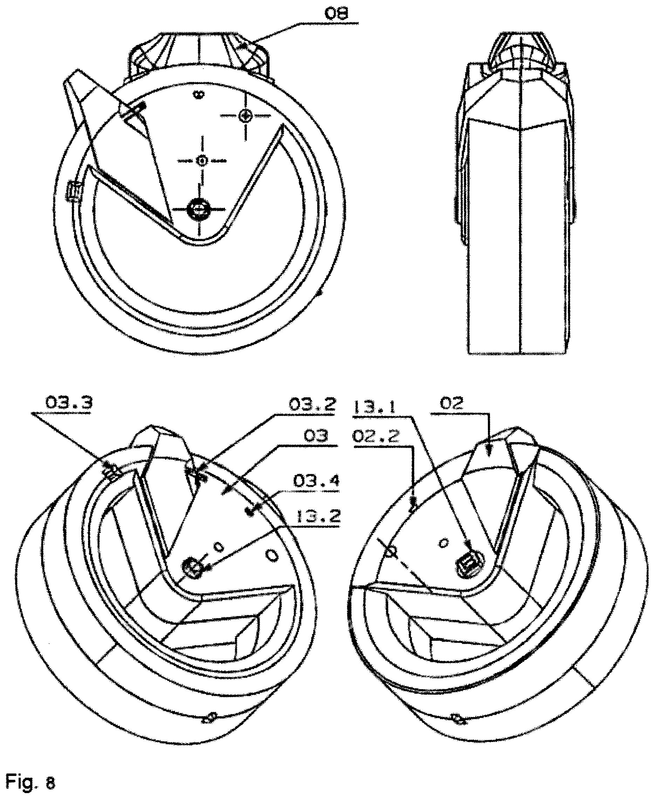

Fig. 8 is a general view of the mechanism enclosed by the housings, also showing the key elements.

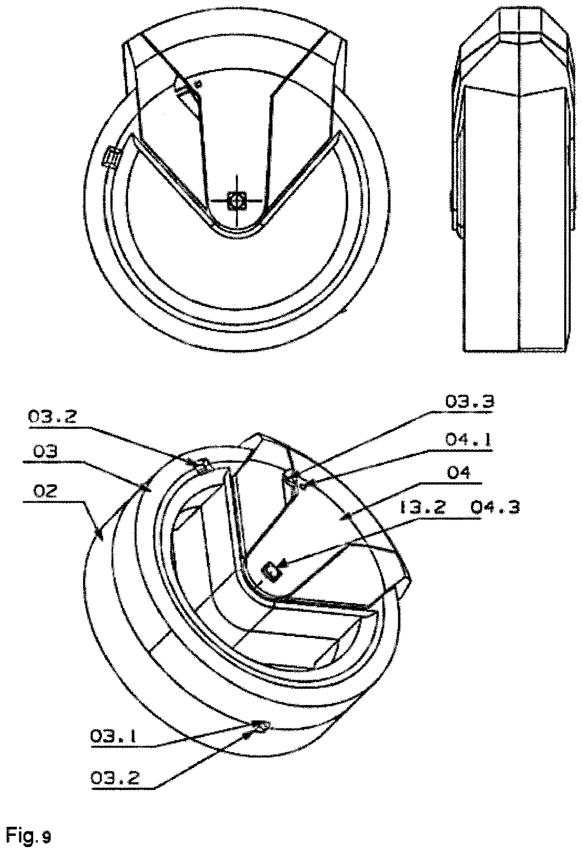

Fig. 9 is an overall view of the entire inhaler in a position with the slide closed, also showing the key elements.

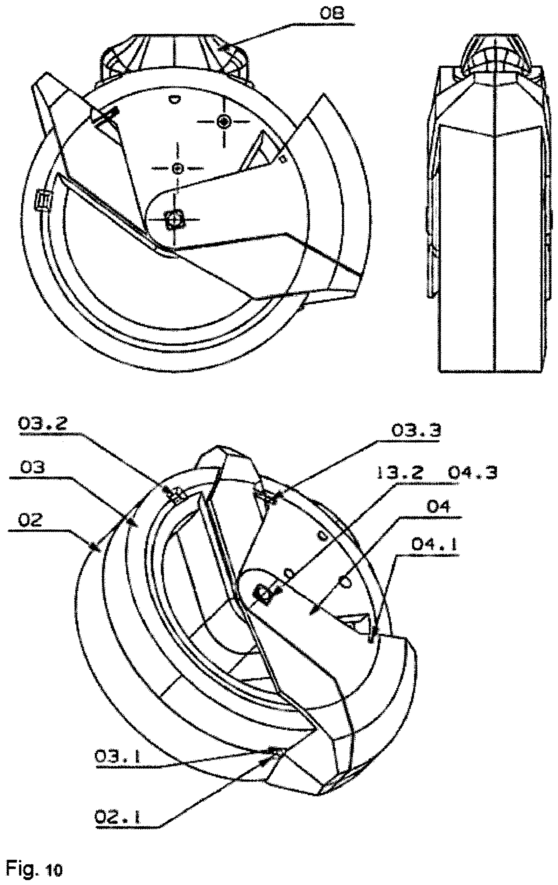

Fig. 10 is an overall view of the entire inhaler in a position with the slide opened, also showing the key elements.

List of reference numerals in the drawings:
01 Blister pack
01.1 blister pack pocket
01.2 lidding layer
01.2.1 lidding layer end
01.3 layer of pockets
01.3.1 end of the layer of pockets
02 lower housing
02.1 stop
02.2 air channels
03 upper housing
03.1 stop
03.2 slide locking lever
03.3 dose window
03.4 air channels
04 slide
04.1 locking lever hook
04.2 upper driving roller slot
04.3 lower driving roller slot
05 body
05.1 air channels
05.2 body latch
06 lower conveyor
06.1 blister pack pocket
06.2 gear wheel
06.3 upper conveyor wedge slot
06.4 blister pack mounting slit
07 upper conveyor
07.1 blister pack pocket
07.2 mounting wedges
07.3 blister pack mounting slit
07.4 air channel
08 mouthpiece
08.1 air channel
09 latch
09.1 latch wings
10 rotor hook
10.1 pin
11 rotor wheel
12 rotor
12.1 rotor wings
13 driving roller
13.1 lower catcher
13.2 upper catcher
14 intermediate wheel
15 latch drum
16 latch wheel

According to the present embodiment, the operating mechanism of the inhaler involves simultaneous movement of two geometrically identical blister packs 01 by a specified step determined by the distance between the pockets 01.1 with the medicament in each blister pack strip, and the positioning of the pockets centrally to the axes of air channels 05.1 in the body 05. Each of the two blister packs 01 is mounted in their corresponding conveyors 06 and 07, coupled with each other by means of gear wheels 06.2, 09, 11, 13, latch elements 09.1, 05.2 connected to each other so as to prevent the movement of only one blister pack, and so that both blister packs would move by a constant identical value. The individual parts of the mechanism except the upper conveyor 07 of the second blister pack are seated in a lower housing 02 and covered by a body 05. The conveyor of the second blister pack 07 is placed above the body, and the whole is covered by an upper housing 03.

Each of the blister packs 01 has 30 pockets with the medicament.

The blister packs 01 are mounted on two separate conveyors: lower 06 and upper 07, having the shape of rings placed one above the other and connected to each other by means of a system of slots 06.3 and wedges 07.2. Each of the blister pack pockets 01.1 has its corresponding, fixed pocket 06.1 and 07.1 in their corresponding conveyors 06 and 07. The lidding layer of the blister pack 01.2 is torn right before the air chamber 05.1 of the body 05, each time exposing one pocket from each blister pack. The lidding layer travels in a fixed position on the conveyor, attached to a mounting slit 06.4 in the lower conveyor 06 and correspondingly 07.3 in the upper conveyor (07), rotating with each consecutive dosing by a constant angle of rotation resulting from the position of the pockets in the blister packs. The pocket foil 01.3 is mounted by its end 01.3.1 on the pin 10.01 of a rotor hook 10. The pocket foil 01.3 is wound onto the rotor 12 by a constant value, which is adjusted by flexible wings 12.1 and the selected gear ratio of the gear wheels of the mechanism. The rotor 12 is seated on the rotor hook 10, which is in turn seated in a rotor wheel 11. The rotor gear wheel 11 and the conveyor 06 are coupled with each other via an intermediate wheel 04, a latch wheel 16, a latch 09, a latch drum 15, and they are driven by a driving roller 13. The driving roller 13, via the lower 13.1 and upper catcher 13.2, transmits the rotation from a slide 04 connected to the driving roller by recesses 04.2 and 04.3. The slide 04 serves two functions: 1 - it protects the mouthpiece 08 against dirt, 2 - it serves as a lever for activating the dosing mechanism. In order to prevent accidental/unauthorised opening of the slide 04, which could result in releasing a dose of the medicament, in the presented solution, a locking lever 03.2 has been introduced as an elastic element in the upper housing 03. In order to activate the dosing mechanism, it is necessary to press the lever 03.2 along with simultaneous opening of the slide 04. The slide 04 is to be rotated by a constant angle of rotation, resulting from the applied gear ratio of the gear wheels, up to the stops 03.1 and 02.1 positioned on the upper 03 and lower housing 02, respectively. Via the gear wheels, the rotation of the housing will allow for rotating the conveyors 06 and 07 and rotors 12, to which elements separate layers of blister packs 01 are attached with an angle allowing for tearing the blister packs 01 and positioning the open pockets 01.1 with the medicaments opposite the air channels 05.1 of the body 05. In order to gain access to the healing content of the open pockets in the blister packs, the patient breathes in, with their mouth tightly contacting the mouthpiece 08. This results in sucking the air into the air channels 05.1, and in flow through the open pockets of the blister packs 01.1. The air channels 05.1 of the body are connected to the air channels in the upper 03.4 and lower housing 02.2, and, via channels 07.4 formed by connecting the conveyors 06 and 07 to each other, they are connected to the channel 08.1 of the mouthpiece 08. The suction of air causes the powdered medicament present in the open pockets 01.1 to transform into an aerosol and flow to a place where it mixes with itself 07.4, and then to the outlet of the mouthpiece 08. The mixing of powder takes place during the transport of powder from each open pocket. Upon administering the medicament, the patient covers the mouthpiece 08 with the slide 04. Because of the latches 09.1 and 05.2, the rotation of the rotors 12 and conveyors 06 and 07 is blocked, and it is only possible to cover the mouthpiece 08, without setting the entire mechanism in a return motion. In this embodiment, the device is provided with a dose counter placed on the conveyor 07. Each of the slots on the conveyor is described by a printed number. The number is visible through a window 03.3 in the upper housing 03.

The inhaler according to the invention provides invariability of the dose size, also minimising the risks of accidental exposure of the mouthpiece, and results in a need to use minor force in order to activate the mechanism, ensuring smoothness of operation.

## Claims

1. An inhaler for administering a medicament in the form of dry powder from blister packs seated on a conveyor, wherein the lidding layer of the blister pack is torn before an air chamber, whereas the medicament in the form of powder is transported by breathing in through the mouth contacting a mouthpiece, causing suction of the air and flow through an open blister pack pocket, **characterised in that** the administration of the medicament involves simultaneous movement of two geometrically identical blister packs (01) by a specified step determined by the distance between the pockets (01.1) with the medicament in each blister pack strip, and positioning the pockets centrally to the axes of air channels (05.1) in the body (05), each of the two blister packs (01) being mounted in their corresponding conveyors (06) and (07), coupled with each other by means of gear wheels (06.2), (09), (11), (13), latch elements (09.1), (05.2) connected to each other so as to prevent the movement of only one blister pack, and so that both blister packs would move by a constant identical value.

2. The inhaler for administering a medicament in the form of dry powder according to claim 1, **characterised in that** the individual parts of the mechanism except the upper conveyor (07) of the second blister pack are seated in a lower housing (02) and covered by a body (05), the conveyor of the second blister pack (07) being placed above the body and the whole being covered by an upper housing (03), the blister packs (01) being mounted on two separate conveyors: lower (06) and upper (07), having the shape of rings placed one above the other and connected to each other by means of a system of slots (06.3) and wedges (07.2).

3. The inhaler for administering the medicament in the form of dry powder according to claim 1 or 2, **characterised in that** each of the blister pack pockets (01.1) has its corresponding, fixed pocket (06.1) and (07.1) in their corresponding conveyors (06) and (07), the lidding layer of the blister pack (01.2) being torn right before the air chamber (05.1) of the body (05), each time exposing one pocket from each blister pack, the lidding layer travelling in a fixed position on the conveyor, attached to a mounting slit (06.4) in the lower conveyor (06) and correspondingly (07.3) in the upper conveyor (07), rotating with each subsequent dosing by a constant angle of rotation, whereas the pocket foil (01.3) is mounted by its end (01.3.1) on the pin (10.01) of a rotor hook (10), the pocket foil (01.3) becoming wound on the rotor (12) by a constant value adjusted by flexible wings (12.1) and the selected gear ratio of the gear wheels, whereas the rotor (12) is seated on the rotor hook (10), which in turn is seated in a rotor wheel (11), the rotor gear wheel (11) and the conveyor (06) being coupled with each other via an intermediate wheel (04), a latch wheel (16), a latch (09), a latch drum (15), and they are driven by a driving roller (13), the driving roller (13), via the lower (13.1) and upper catcher (13.2), transmitting the rotation from a slide (04) connected to the driving roller by recesses (04.2) and (04.3), the air channels (05.1) of the body being connected to the air channels in the upper (03.4) and lower housing (02.2), and, via channels (07.4) formed by connecting the conveyors (06) and (07) to each other, they are connected to the channel (08.1) of the mouthpiece (08).

4. The inhaler for administering a medicament in the form of dry powder according to any of the preceding claims, **characterised in that** each of the blister packs (01) has 30 pockets with the medicament.

5. The inhaler for administering a medicament in the form of dry powder according to claim 2 or 3, **characterised in that** a locking lever (03.2) has been introduced in the upper housing (03) as an elastic element for activating the dosing mechanism.

6. The inhaler for administering a medicament in the form of dry powder according to any of the preceding claims, **characterised in that** a dose counter is placed on the conveyor (07), each of the slots on the conveyor being described by a printed number, the number being visible through a window (03.3) in the upper housing (03).

7. The inhaler for administering a medicament in the form of dry powder according to any of the preceding claims, **characterised in that** it uses a high gear ratio on the gear wheels, causing the force needed to activate the mechanism to be minimised, wherein the arrangement of the blister packs one above the other allows for minimising the number of parts in the mechanism.
